# FASCICULE DE BREVET EUROPEEN

(11) **EP 1 778 721 B1**
(45) Date de publication et mention de la délivrance du brevet: **09.04.2008**
(21) Numéro de dépôt: 05793254.3
(22) Date de dépôt: 27.07.2005
(51) Int. Cl.: C07K 14/16, C07K 16/10, G01N 33/569

(54) **PEPTIDES VIH-1 MODIFIES ET LEUR UTILISATION EN DETECTION D'ANTICORPS ANTI-VIH**
MODIFIZIERTE HIV-1-PEPTIDE UND IHRE VERWENDUNG BEIM NACHWEISEN VON ANTI-HIV-ANTIKÖRPERN
MODIFIED HIV-1 PEPTIDES AND USE THEREOF IN THE DETECTION OF ANTI-HIV ANTIBODIES

(30) Priorité: 06.08.2004 FR 0408733
(43) Date de publication de la demande: 02.05.2007
(73) Titulaire: Bio-Rad Pasteur, 92430 Marnes-la-Coquette (FR)
(72) Inventeur: GADELLE, Stéphane, F-91430 Vauhallan (FR); RIEUNIER, François, F-78390 Bois d'Arcy (FR)
(74) Mandataire: Colombet, Alain André
(86) Numéro de dépôt international: PCT/FR2005/001969
(87) Numéro de publication internationale: WO 2006/024752

(56) Documents cités:
- EP-A- 0 326 490
- WO-A-98/45323
- US-A1- 2004 072 267

### SEQUENCE LISTING

<110> Bio-Rad Pasteur
<120> Peptides VIH-1 modifiés et leur utilisation en détection d'anticorps anti- VIH
<130> BET 05P0796
<150> FR 0408733
   <151> 2004-08-06
<160> 11
<170> PatentIn version 3.1
<210> 1
   <211> 35
   <212> PRT
   <213> Human immunodeficiency virus type 1
<220>
   <221> DISULFID
   <222> (20)..(26)
   <223>
<400> 1
   Arg Ile Leu Ala Val Glu Arg Tyr Leu Lys Asp Gln Gln Leu Leu Gly
   1 5 10 15
   Ile Trp Gly Cys Ser Gly Lys Leu Ile Cys Thr Thr Ala Val Pro Phe
   20 25 30
   Asn Ala Ser
   35
<210> 2
   <211> 33
   <212> PRT
   <213> Human immunodeficiency virus type 1
<220>
   <221> DISULFID
   <222> (20)..(26)
   <223>
<400> 2
   Arg Ile Leu Ala Val Glu Arg Tyr Leu Lys Asp Gln Gln Leu Leu Gly
   1 5 10 15
   Ile Trp Gly Cys Ser Gly Lys Leu Ile Cys Thr Thr Ala Val Pro Phe
   20 25 30
   Asn
<210> 3
   <211> 33
   <212> PRT
   <213> Human immunodeficiency virus type 1
<220>
   <221> DISULFID
   <222> (20)..(26)
   <223>
<400> 3
   Arg Ile Leu Ala Val Glu Arg Tyr Leu Lys Asp Gln Gln Leu Leu Gly
   1               5                   10                  15
   Ile Trp Gly Cys Ser Gly Lys Leu Ile Cys Thr Thr Ala Val Pro Gly
               20                  25                  30
   Asn
<210> 4
   <211> 35
   <212> PRT
   <213> Human immunodeficiency virus type 1
<220>
   <221> DISULFID
   <222> (20)..(26)
   <223>
<400> 4
   Arg Ile Leu Ala Val Glu Arg Tyr Leu Lys Asp Gln Gln Leu Leu Gly
   1               5                   10                  15
   Ile Trp Gly Cys Ser Gly Lys Leu Ile Cys Thr Thr Ala Val Pro Gly
               20                  25                  30
   Asn Ala Ser
           35
<210> 5
   <211> 35
   <212> PRT
   <213> Human immunodeficiency virus type 1
<220>
   <221> DISULFID
   <222> (20)..(26)
   <223>
<220>
   <221> MISC_FEATURE
   <222> (32)..(32)
   <223> Xaa = Phe or Gly
<220>
   <221> MISC_FEATURE
   <222> (34)..(35)
   <223> Xaa Xaa = absent or Ala Ser
<400> 5
   Arg Ile Leu Ala Val Glu Arg Tyr Leu Lys Asp Gln Gln Leu Leu Gly
   1               5                   10                  15
   Ile Trp Gly Cys Ser Gly Lys Leu Ile Cys Thr Thr Ala Val Pro Xaa
               20                  25                  30
   Asn Xaa Xaa
           35
<210> 6
   <211> 35
   <212> PRT
   <213> Human immunodeficiency virus type 1
<220>
   <221> MISC_FEATURE
   <222> (32)..(32)
   <223> Xaa = Phe or Gly
<220>
   <221> MISC_FEATURE
   <222> (34)..(35)
   <223> Xaa Xaa = absent or Ala Ser
<400> 6
   Arg Ile Leu Ala Val Glu Arg Tyr Leu Lys Asp Gln Gln Leu Leu Gly
   1               5                   10                  15
   Ile Trp Gly Cys Ser Gly Lys Leu Ile Cys Thr Thr Ala Val Pro Xaa
               20                  25                  30
   Asn Xaa Xaa
           35
<210> 7
   <211> 35
   <212> PRT
   <213> Human immunodeficiency virus type 1
<220>
   <221> DISULFID
   <222> (20)..(26)
   <223>
<400> 7
   Arg Ile Leu Ala Val Glu Arg Tyr Leu Lys Asp Gln Gln Leu Leu Gly
   1 5 10 15
   Ile Trp Gly Cys Ser Gly Lys Leu Ile Cys Thr Thr Ala Val Pro Trp
   20 25 30
   Asn Ala Ser
   35
<210> 8
   <211> 21
   <212> PRT
   <213> Human immunodeficiency virus type 1
<400> 8
   Arg Ile Leu Ala Val Glu Arg Tyr Leu Lys Asp Gln Gln Leu Leu Gly
   1 5 10 15
   Ile Trp Gly Cys Ser
   20
<210> 9
   <211> 26
   <212> PRT
   <213> Human immunodeficiency virus type 1
<400> 9
   Arg Ile Leu Ala Val Glu Arg Tyr Leu Lys Asp Gln Gln Leu Leu Gly
   1 5 10 15
   Ile Trp Gly Cys Ser Gly Lys Leu Ile Cys
   20 25
<210> 10
   <211> 35
   <212> PRT
   <213> Human immunodeficiency virus type 1
<400> 10
   Arg Ile Leu Ala Val Glu Arg Tyr Leu Lys Asp Gln Gln Leu Leu Gly
   1               5                   10                  15
   Ile Trp Gly Cys Ser Gly Lys Leu Ile Cys Thr Thr Ala Val Pro Trp
               20                  25                  30
   Asn Ala Ser
           35
<210> 11
   <211> 7
   <212> PRT
   <213> Human immunodeficiency virus type 1
<400> 11
   Cys Ser Gly Lys Leu Ile Cys
   1               5

## Description

L'invention concerne des peptides synthétiques utilisables dans les essais immunologiques de détection des infections dues aux virus VIH-1, leur procédé de préparation, des compositions et des trousses contenant de tels peptides, l'utilisation de tels peptides à des fins diagnostiques ainsi que des procédés d'essai immunologique les mettant en oeuvre en vue de la détection d'anticorps anti virus VIH.

Le rétrovirus du type VIH-1 (Virus de l'Immunodéficience Humaine de type 1 ou HIV-1, en anglais) est connu comme étant historiquement le premier agent responsable du SIDA chez l'homme. Aujourd'hui, on distingue parmi les virus du type VIH-1, les virus VIH-1 du groupe M (groupe majeur) et les virus VIH-1 du groupe O (groupe minoritaire, appelé initialement sous-type O) qui en diffèrent, entre autres, génomiquement et immunologiquement. On connaît depuis 1986 (Clavel et al., Science, vol. 233, pp. 343-346, 18 July 1986) un deuxième type de virus responsable du SIDA chez l'homme, dénommé VIH-2 (HIV-2, en anglais).

Pour des raisons de commodité d'expression, les virus VIH-1 du groupe M seront, dans le texte qui suit, désignés par l'expression « VIH-1 M » et les virus VIH-1 du groupe O, par l'expression « VIH-1 O ».

Le rétrovirus du type VIH-1 a été découvert initialement sous forme de 3 isolats distincts. Ces isolats sont dénommés LAV, HTLV-III, et ARV, isolat quelquefois dénommé aussi ARV-2. On pourra se référer aux articles Barré-Sinoussi et al. (Science 20 May 1983, 220, pp.868-871); Popovic et al. (Science 4 May 1984, 224, pp.497-500) ; Gallo et al. (Science 4 May 1984, 224, pp. 500-503) et Levy et al. (Science 24 August 1984, 225, pp.840-842) qui font état de la découverte de ces isolat. Ces 3 isolats font tous partie, aujourd'hui, de la catégorie des virus VIH-1 M. Les virus VIH-1 O ont été décrits bien plus tard, dès 1990, et leurs isolats ont eu des désignations différentes telles que HIV-3 ou ANT 70 (demande de brevet européen EP 345 375 et De Leys et al., Journal of Virology, March 1990, Vol. 64, No.3, p. 1207-1216), ou, plus tard, MVP5180/91 (Gürtler et al., Journal of Virology, March 1994, Vol. 68, No.3, p. 1581-1585 ; demande de brevet EP 0 591 914), etc...D'autres isolats du groupe O, tels que les HIV-1_{VAU}, HIV-1_{DUR}, MVP2901/94, etc... ont, depuis, été décrits.

La séquence des premiers isolats du rétrovirus VIH-1 M a été élucidée et publiée au début de l'année 1985 : on pourra se référer aux articles Wain-Hobson et al. (Cell, January 1985, 40, pp. 9-17) ; Ratner et al. (Nature, 24 January 1985, 313, pp. 277-284) ; et Sanchez-Pescador et al. (Science 1 February 1985, 227, pp.484-492). Les virus LAV, HTLV-III et ARV/ARV-2 ont depuis été reconnus comme étant des variants du même virus du SIDA, maintenant connu sous le nom de VIH-1 ou HIV-1 (pour Human Immunodeficiency Virus) (Ratner et al., Nature, vol. 313, 21 February 1985, pp. 636-637).

Les premiers essais de diagnostic *in-vitro* de l'infection par le VIH-1 M initiés en 1984-1985 ont été réalisés par immunoessai et visaient la détection de la présence d'anticorps anti-VIH-1 M dans des échantillons biologiques humains tels que le sérum ou le plasma. Ces premiers immunoessais de détection d'anticorps anti VIH-1 M faisaient usage de lysat viral comme antigène cible pour capturer les anticorps recherchés (ce sont les immunoessais dits de première génération). Comme ils donnaient lieu quelquefois à des résultats faussement négatifs et/ou à des résultats faussement positifs en raison de l'insuffisant degré de pureté de la préparation antigénique qu'ils utilisaient, on a alors eu recours au génie génétique pour produire des antigènes mieux contrôlés, plus homogènes et qui se sont avérés plus sensibles et plus spécifiques. On peut citer, par exemple, les travaux réalisés par plusieurs équipes sur diverses formes d'antigène de la glycoprotéine transmembranaire d'enveloppe, la gp41, du VIH-1 M et les immunoessais les mettant en oeuvre, travaux rapportés dans les articles Chang et al. (Science, 228, 5 April 1985, pp. 93-96) ; Crowl et al. (Cell, 41, July 1985, pp. 979-986) ; Chang et al. (BioTechnology, 3, October 1985, pp. 905-909) ; Cabradilla et al. (BioTechnology, 4, February, 1986, pp. 128-133), etc... Ces immunoessais à base d'antigène recombiné ont constitué alors les immunoessais de deuxième génération. Bien qu'apportant un grand progrès, ces nouveaux immunoessais ne permettaient toujours pas de détecter tous les sérums de sujets infectés par le VIH-1 M.

Dans une recherche de sensibilité et de spécificité toujours plus approfondie, certaines équipes se sont tournées vers les peptides synthétiques, courts (moins de 50 acides aminés, en général), faciles à produire et à contrôler, et utilisables comme antigènes cibles pour détecter les anticorps anti-VIH-1 M. C'est ainsi que Wang et al. (PNAS, Vol. 83, pp. 6259-6163, August 1986) décrivent l'emploi, comme antigène de capture des anticorps, d'un peptide de la gp41 d'un VIH-1 M, de séquence RILAVERYLKDQQLLGIWGC₆₀₃S (SEQ ID N° 8).

De la même manière, la demande de brevet WO86/06414 au nom de Genetic Systems Corporation décrit une série de peptides courts, dont certains dérivés de la gp41 du VIH-1 M_{BRU}, comme le peptide (X) (39), qui correspond à la séquence très voisine RILAVERYLKDQQLLGIWGC₆₀₃SGKLIC₆₀₉ (SEQ ID N° 9).

Le brevet US 4,879,212 (Wang et al.) décrit un peptide de la gp41 du VIH-1 M un peu plus long (35 acides aminés), de séquence :
RILAVERYLKDQQLLGIWGC₆₀₃SGKLIC₆₀₉TTAVPWNAS (SEQ ID N° 10).

Les peptides de Wang et al. et ceux de la demande de brevet WO86/06414 confèrent une très grande sensibilité et une très grande spécificité aux nouveaux immunoessais à base de peptides, dits immunoessais de troisième génération. De nombreux auteurs ont suivi la voie des peptides courts pour la mise au point de nouveaux réactifs et, depuis lors, de nombreuses trousses (kits) de détection d'anticorps anti-VIH commerciales en contiennent.

L'équipe de John W. Gnann, à la clinique Scripps de La Jolla, en Californie, a même identifié dans le peptide (X) (39) ci-dessus un épitope diagnostiquement très important, du fait qu'il est à la fois très immunoréactif et très spécifique du VIH-1 M : c'est l'épitope immunodominant - de séquence WGC₆₀₃SGKLIC₆₀₉ - de la gp41 du VIH-1 M (Gnann et al., Journal of Virology, August 1987, p. 2639-2641; Gnann et al., Science, vol. 237, 11 September 1987, pp. 1346-1349). Gnann et al. ont émis l'idée que, dans cet épitope immunodominant, la formation d'un pont disulfure entre les deux cystéines C₆₀₃ et C₆₀₉ pourrait jouer un rôle clef dans la conformation antigénique de l'épitope, en favorisant potentiellement la création d'une structure cyclique.

Peu après, les demandes de brevet WO89/03844 au nom de Ferring AB et EP 0 326 490 A2 au nom de IAF Biochem International décrivaient des peptides de la gp41 du VIH-1 M portant « l'épitope de Gnann et al. » sous forme volontairement cyclisée par un pont disulfure entre les deux cystéines C₆₀₃ et C₆₀₉ que l'on peut représenter de manière générale, simplifiée et symbolisée par la formule arbitraire suivante : Elles confirmaient l'intuition pionnière de Gnann et al.

La demande EP 0 326 490 A2 décrivait, entre autres, le peptide 35mer de la gp41 du VIH-1 M du brevet US 4,879,212 (Wang et al.), mais sous forme cyclisée et de séquence (I):

L'amélioration apportée par de tels peptides ainsi cyclisés reste toutefois sujette à controverse, ces derniers peptides ne permettant pas la détection suffisamment précoce de certains échantillons de séroconversion du VIH-1 M. C'est ainsi, en particulier, que les performances du peptide cyclique de séquence (I) ci-dessus sont encore insatisfaisantes en sensibilité, vu que ce peptide ne permet pas de détecter tous les échantillons de séroconversion positifs en anticorps anti VIH-1 M, et que, de plus, il pose des problèmes, de rendement de synthèse et de solubilité.

En bref, quel que soit le peptide de la gp41 que l'on utilise comme antigène cible en détection des séroconversions anti VIH-1 M, il reste encore quelques échantillons, faiblement positifs, qui ne sont pas détectés par ces immunoessais de troisième génération. Il existe donc un besoin, dans l'art, de réactifs améliorés, solubles, et utilisables en détection d'échantillons positifs en anticorps anti VIH-1 M. II existe, en particulier, un besoin de réactifs de sensibilité améliorée permettant de détecter encore plus précocément les séroconversions. Il est en effet crucial en transfusion sanguine de détecter au plus tôt tout échantillon infecté par le VIH-1 M. Gagner ne serait-ce qu'une semaine de précocité de détection est très important pour éviter, par exemple, de transmettre le virus à un sujet transfusé.

Les auteurs de la présente invention ont commencé par chercher à fabriquer un peptide proche du peptide cyclique de séquence (I), mais plus aisément, i.e. avec un meilleur rendement. Parmi les diverses approches possibles, l'une consistait à simplement réduire la taille du peptide, une autre, à remplacer les résidus d'acides aminés susceptibles de générer une baisse des rendements de synthèse (problèmes de solubilité, de couplage, de réactions secondaires ...). Une autre approche possible consistait à allonger le peptide à l'aide d'acides aminés hydrophiles, pour essayer de le rendre plus soluble, etc...

Les inventeurs ont remplacé le résidu tryptophane en position 615 par différents résidus pour obtenir un peptide linéaire (non cyclisé entre les deux résidus cystéine) de séquence (II) suivante :
RILAVERYLKDQQLLGIWGC₆₀₃SGKLIC₆₀₉TTAVPX₆₁₅Naa₁aa₂ (II) (SEQ ID N° 6)
où
X représente un acide aminé choisi dans le groupe constitué par F et G,
et
où les deux acides aminés aa₁ et aa₂ soit sont absents, soit représentent
aa₁ : l'alanine
aa₂ : la sérine.

Comme c'est connu de l'homme du métier, les lettres F et G symbolisent les acides aminés phénylalanine (F) et glycine (G).

Par oxydation directe des deux cystéines sur des peptides de séquence (II), les inventeurs ont ensuite préparé des peptides cyclisés répondant à la formule (III) suivante : où
X représente un acide aminé choisi dans le groupe constitué par F et G,
et
où les deux acides aminés aa₁ et aa₂ soit sont absents, soit représentent
aa₁ : l'alanine
aa₂ : la sérine.
Ils ont, en particulier, synthétisé les peptides cyclisés ci-après, désignés respectivement SEQ ID N°1, N°2, N°3, et N°4 :

De façon surprenante et totalement inattendue, vu que, d'une part, pour certains peptides on raccourcissait la séquence longue initialement de 35 acides aminés, au risque de perdre ainsi un épitope fonctionnel, et que, d'autre part, on générait des séquences partiellement artificielles, non rencontrées dans les souches virales naturelles, les inventeurs ont constaté qu'en modifiant ainsi le peptide cyclique de formule (I) de l'art antérieur, qui ne détectait pas tous les échantillons de séroconversion positifs en anticorps anti VIH-1 M, on obtenait de nouveaux peptides, les peptides de séquence (III), qui étaient capables de détecter tous ces échantillons.

La présente invention a donc pour objet un peptide cyclique de la gp41 du virus VIH-1 de séquence (III) : où
X représente un acide aminé choisi dans le groupe constitué par F et G,
et
où les deux acides aminés aa₁ et aa₂ soit sont absents, soit représentent
aa₁ : l'alanine
aa₂ : la sérine.

La présente invention a aussi pour objet une composition, en particulier une composition antigénique, comprenant un peptide de séquence (III).

La présente invention a encore pour objet un peptide de la gp41 du virus VIH-1 de séquence (III) choisi dans le groupe constitué par les peptides suivants :

La présente invention a aussi pour objet une composition, en particulier une composition antigénique, comprenant un peptide de séquence (III) choisi dans le groupe constitué par les peptides SEQ ID N°1 à 4 vus plus haut.

Par « composition » dans le cadre de la présente invention, il faut entendre toute association liquide ou solide de plusieurs composants moléculaires. Certaines compositions, ou mélanges, sont constituées par des solutions aqueuses, liquides ou solides, comprenant un ou plusieurs composants chimique d'intérêt. Une telle solution aqueuse peut être constituée d'un tampon, de pH généralement proche de la neutralité (de préférence entre pH 5 et pH 9), de préférence en association avec un ou plusieurs protides (i.e. protéines, polypeptides, oligopeptides et/ou acides aminés) utiles pour le but recherché. Parmi les tampons bien connus de l'homme du métier, on peut citer, à titre non limitatif, les tampons phosphate, carbonate, Tris, borate, etc.. Parmi les protéines utilisables dans les tampons, on peut citer, entre autres, l'albumine sérique bovine (BSA), etc.. Un agent conservateur, tel que l'azoture de sodium, par exemple, est souvent ajouté à de telles compositions, ainsi qu'éventuellement un détergent.

Par « composition antigénique» dans le cadre de la présente invention, il faut entendre toute composition visée ci-dessus contenant au moins un peptide selon l'invention (de séquence (III)) sous une forme telle que ce dernier conserve substantiellement sa réactivité antigénique initiale, c'est à dire sa capacité à être reconnu par des anticorps anti VIH. De telles compositions antigéniques peuvent incorporer aussi un antigène du VIH-2 et/ou un antigène du VIH-1 O, maintenus immunoréactifs.

De telles compositions antigéniques peuvent servir au diagnostic *in-vitro*, dans un échantillon de fluide biologique humain, d'une infection par un VIH, et en particulier, mais non exclusivement, à la détection d'anticorps anti VIH, selon toute forme de protocole d'immunoessai en soi connue de l'homme du métier (voir, entre autres, ci-après la section « Description plus détaillée de l'invention », au paragraphe 4).

De telles compositions antigéniques peuvent être incorporées sous forme liquide ou sèche dans des trousses de diagnostic *in-vitro* d'une infection par VIH, en particulier dans des trousses de détection d'anticorps anti VIH. Ces compositions antigéniques peuvent être immobilisées sur une phase solide, ou incorporées dans un antigène de détection, tel qu'un antigène marqué, notamment marqué par une enzyme, etc.. selon des méthodes en soi connues de l'homme du métier.

La présente invention a de plus pour objet un procédé de préparation d'un peptide de séquence (III), notamment d'un peptide choisi dans le groupe constitué par les peptides SEQ ID N°1, SEQ ID N°2, SEQ ID N°3 et SEQ ID N°4.

La présente invention a en outre pour objet un procédé de préparation d'une composition comprenant un peptide de séquence (III), notamment un peptide choisi dans le groupe constitué par les peptides SEQ ID N°1, SEQ ID N°2, SEQ ID N°3 et SEQ ID N°4.

La présente invention a encore pour objet un procédé de détection d'anticorps anti VIH dans un échantillon biologique comprenant
a) la mise en contact de l'échantillon biologique soit avec un peptide de la gp41 du virus VIH-1 de séquence (III), notamment un peptide choisi dans le groupe constitué par les peptides SEQ ID N°1, SEQ ID N°2, SEQ ID N°3 et SEQ ID N°4, et/ou une composition comprenant un tel peptide ; soit avec une combinaison de peptides de séquence (III) ;
b) l'incubation du mélange dans des conditions permettant la formation de complexes antigène - anticorps ; et
c) la révélation et la détection des complexes antigènes-anticorps formés par un moyen de détection, constitué éventuellement par un antigène VIH-1, pouvant le cas échéant comprendre un peptide de séquence (III) selon l'invention, marqué, capable de se lier à un anticorps anti VIH-1 capturé.

La présente invention a encore pour objet le procédé de détection d'anticorps anti VIH précédent dont l'étape a) comprend la mise en contact de l'échantillon biologique avec un mélange constitué d'au moins un peptide de la gp41 de séquence (III) selon l'invention, et d'un antigène du VIH-2 et/ou d'un antigène du VIH-1 O.

La présente invention a aussi pour objet une trousse de détection d'anticorps anti VIH dans un échantillon biologique contenant un peptide de la gp41 du virus VIH1 de séquence (III), notamment un peptide choisi dans le groupe constitué par les peptides SEQ ID N°1, SEQ ID N°2, SEQ ID N°3 et SEQ ID N°4, ou une composition comprenant un tel peptide, éventuellement en association avec un antigène du VIH-2 et/ou un antigène du VIH-1 O.

La présente invention a encore pour objet l'utilisation d'au moins un peptide de la gp41 du virus VIH1 de séquence (III), notamment un peptide choisi dans le groupe constitué par les peptides SEQ ID N°1, SEQ ID N°2, SEQ ID N°3 et SEQ ID N°4, éventuellement en association avec un antigène du VIH-2 et/ou un antigène du VIH-1 O, en vue de la détection d'anticorps anti virus VIH.

### Description plus détaillée de l'invention

### 1. Les échantillons biologiques :

Par l'expression « échantillon biologique » on entend, dans le cadre de la présente invention, un échantillon de tout fluide corporel humain tel que le sang, le sérum, le plasma, la salive, les larmes, le sperme, le liquide céphalorachidien et tout autre fluide corporel susceptible de contenir des anticorps anti-VIH.

### 2. Préparation des peptides selon l'invention :

De manière préférentielle, car plus pratique, mais non exclusive, les peptides de séquence (III) selon l'invention sont produits par les techniques classiques bien connues de l'homme du métier. On peut citer comme exemple la synthèse de type Merrifield (Merrifield, J. Am. Chem. Soc. 85, pp. 2149-2154, 1963). On peut également utiliser la synthèse de type « Fmoc » (9-fluorénylméthyloxy carbonyl) qui est avantageuse pour des raisons de pureté, de spécificité antigénique, d'absence de produits secondaires non désirés et pour sa facilité de mise en oeuvre.

La synthèse de peptides peut être automatisée à l'aide de synthétiseurs, par exemple, du type du synthétiseur "Pioneer" de Perspective, du synthétiseur "433A" de ABI (Applied Biosystems Inc.) ou du synthétiseur « Symphony » de Rainin.

Les peptides peuvent également être obtenus par synthèse en phase homogène selon des techniques en soi connues de l'homme du métier.

La cyclisation de peptides linéaires en vue d'obtenir des peptides cyclisés de formule (III) selon l'invention peut se réaliser de diverses manières en soi connues de l'homme du métier, en particulier selon l'une des méthodes exposées dans les demandes de brevet WO89/03844 (étape d'oxydation chimique à l'iode en solution méthanolique) et EP 0 326 490 A2 (méthode au ferricyanure de potassium).

Par des méthodes en soi connues de l'homme du métier, les peptides selon l'invention peuvent être éventuellement incorporés dans des tampons, en présence de protéines, polypeptides, oligopeptides, ou acides aminés et d'autres composés chimiques utiles pour constituer des compositions antigéniques selon l'invention.

### 3. Autres antigènes VIH utilisables dans le procédé de détection d'anticorps anti VIH et dans la trousse de détection d'anticorps anti VIH selon l'invention :

A titre d'antigène du VIH-2, on peut utiliser une variété d'antigènes, de manière préférée la gp140 ou la glycoprotéine transmembranaire gp36 du VIH-2, de manière plus préférée un peptide contenant au moins l'épitope immunodominant (de la gp36) de Gnann et al., c'est à dire comprenant au moins les acides aminés WGCAFRQVC (voir, par exemple l'article Gnann et al., Science, vol. 237, 11 September 1987, pp. 1346-1349 ; l'article Guyader et al., Nature 16 April 1987, 326, pp.662-669, qui décrit la séquence complète du VIH-2_{ROD}, ou la séquence env donnée dans le brevet EP 0 239 425), de manière encore plus préférée un peptide comprenant au moins les acides aminés WGCAFRQVC sous forme cyclisée par un pont disulfure entre les deux cystéines.

A titre d'antigène du VIH-1 O, on peut utiliser une variété d'antigènes, de manière préférée la gp160 ou la gp41 du VIH-1 O. De manière plus préférée, on peut utiliser un peptide contenant l'épitope immunodominant (gp41) de Gnann et al. comprenant, par exemple, au moins les acides aminés WGCKGKLIC (du MVP5180/91, décrits dans la séquence env de la demande de brevet EP 0 591 914, ou séquence obtenable à partir de la banque de données GenBank sous le N° d'accession L20571, voir Gürtler et al., Journal of Virology, March 1994, Vol. 68, No.3, p. 1581-1585). De manière encore plus préférée, on peut utiliser un peptide comprenant au moins les acides aminés WGCKGKLIC sous forme cyclisée par un pont disulfure entre les deux cystéines.

Alternativement, à titre d'antigène du VIH-1 O, on peut utiliser, par exemple, les acides aminés WGCKGKLVC (du HIV-3/ANT70, séquence obtenable à partir de la banque de données GenBank sous le N° d'accession L20587, voir Gürtler et al., Journal of Virology, March 1994, Vol. 68, No.3, p. 1581-1585). De manière encore plus préférée, on peut utiliser un peptide comprenant au moins les acides aminés WGCKGKLVC sous forme cyclisée par un pont disulfure entre les deux cystéines.

### 4. Modes de réalisation du procédé de détection d'anticorps anti VIH selon l'invention :

Pour ce qui concerne des modes de mise en oeuvre possibles du procédé de détection d'anticorps anti VIH selon l'invention, on pourra avantageusement se référer, par exemple, à la revue « A Decade of Development In Immunoassay Methodology » de James P. Gosling, Clinical Chemistry, 36/8, pp. 1408-1427, (1990) qui cite un grand nombre de technologies et variantes disponibles et connues en Immunoessais.

Le procédé de détection d'anticorps anti VIH dans un échantillon biologique peut se réaliser par un quelconque immunoessai, au sens large, c'est à dire un essai immunologique faisant intervenir la formation d'un complexe immun entre au moins un peptide selon l'invention et au moins un anticorps anti-VIH. Les méthodes d'essai immunologique ou immunoessais (immunoassay, en anglais) sont bien connues de l'homme du métier et peuvent être du type EIA (Enzyme Immuno Assay), ELISA (Enzyme Linked Immuno Sorbent Assay), RIA (Radio Immuno Assay), FIA (Fluoro Immuno Assay), etc... en fonction du marqueur et du réactif marqué choisis.

Le terme « marqué » se réfère aussi bien à un marquage direct (par le biais d'enzymes, radioisotopes, fluorochromes, composés luminescents, etc..) qu'à un marquage indirect (par exemple, par le biais d'anticorps eux-mêmes marqués de manière directe ou à l'aide de réactifs d'une « paire d'affinité » marquée, telle que, mais non exclusivement, la paire avidine marquée-biotine, etc..). Le moyen de détection du procédé de détection d'anticorps anti VIH selon l'invention peut, par exemple, mais non exclusivement, être constitué par un antigène VIH marqué, un anticorps anti-immunoglobuline humaine marqué ou toute autre molécule ayant de l'affinité pour une immunoglobuline comme la protéine A ou la protéine G, sous forme marquée, etc... On peut utiliser, comme marqueur, une enzyme, un radioisotope, un fluorochrome, un composé luminescent, etc... que l'on couple de manière en soi connue audit peptide selon l'invention, ou audit anticorps anti-immunoglobuline, ou à toute autre molécule choisie comme moyen de détection (par exemple, la protéine A ou la protéine G).

Dans le cadre de la présente invention, on peut marquer des peptides à l'aide de la peroxydase du raifort selon un protocole en soi connu, comme la technique de Nakane et Kawaoki (J. Histochem. Cytochem. 22, p. 1984 (1974)), ou toute autre technique de couplage connue de l'homme du métier. D'autres enzymes peuvent être utilisées comme la phosphatase alcaline, etc,.

Le procédé de détection d'anticorps anti VIH selon l'invention peut être réalisé en phase solide (essai hétérogène) ou en phase liquide (essai homogène).

La détection des anticorps anti VIH selon l'invention peut revêtir différents protocoles connus de l'homme du métier : protocoles de type compétitif, de type indirect ou encore de type sandwich antigène-anticorps-antigène classique, encore appelée méthode « sandwich double antigène » (Maiolini et al., Journal of Immunological Methods, 20 (1978) 25-34), en un temps ou en deux temps.

Selon un mode de réalisation en phase hétérogène préféré de l'invention, en protocole de type indirect ou encore en méthode « sandwich double antigène », un antigène d'enveloppe du VIH-1 M correspondant à la gp160, à la gp41 ou à un peptide selon l'invention, utilisé, en association ou non avec un antigène VIH-2 et/ou un antigène VIH-1 O, comme antigène de capture des anticorps, est immobilisé sur une phase solide. On peut utiliser, à titre d'exemples non limitatifs de phase solide, des microplaques, en particulier des microplaques de polystyrène, telles que celles commercialisées par la société Nunc, Danemark. On peut également utiliser des particules ou des billes solides, des billes paramagnétiques, telles que celles fournies par Dynal ou Merck-Eurolab (France) (sous la marque Estapor™), ou encore des tubes à essai en polystyrène ou polypropylène, des bandelettes de nitrocellulose, etc...

Selon un mode de réalisation préféré, dans le cadre de la méthode « sandwich double antigène » (Maiolini et al., 1978), un peptide selon l'invention est utilisé marqué comme moyen de détection, i.e. comme antigène de révélation des complexes formés et immobilisés sur la phase solide.

Selon un autre mode de réalisation préféré, dans le cadre du protocole de type indirect, un anticorps anti-immunoglobuline humaine, ou un fragment (Fab, Fab', etc..) immunoréactif de cet anticorps, peut être utilisé marqué comme moyen de détection, i.e. comme anticorps de révélation des complexes formés et immobilisés sur la phase solide.

### 5. Trousses de détection des anticorps anti VIH

Les trousses et réactifs utiles à la détection d'anticorps anti VIH dans un échantillon biologique, conformément au procédé de l'invention, peuvent être fournis pour une mise en oeuvre de l'invention aisée et applicable à de nombreux échantillons biologiques.

Un objet de l'invention est donc constitué par une trousse de détection d'anticorps anti VIH dans un échantillon biologique, comprenant :
- au moins un antigène de capture et/ou de détection, qui est un peptide pouvant être constitué par un peptide de séquence (III) selon l'invention, ou une composition antigénique contenant un tel peptide;
- au moins un moyen de détection des complexes antigène-anticorps formés.

De manière avantageuse, cette trousse peut contenir plusieurs antigènes de capture et/ou de détection.

Comme cela est décrit ci-dessus, l'antigène de capture peut être présenté de manière avantageuse sous une forme immobilisée sur une phase solide, telle qu'une microplaque.

Une trousse de détection d'anticorps anti VIH dans un échantillon biologique préférée comprend :
a) un antigène de capture, qui est un peptide de séquence (III) selon l'invention, ou une composition antigénique contenant un tel peptide, ledit antigène de capture étant immobilisé sur une microplaque;
b) un antigène de détection, marqué par une enzyme.

Une autre trousse de détection d'anticorps anti VIH dans un échantillon biologique préférée comprend
a) un antigène de capture, ledit antigène de capture étant immobilisé sur une microplaque;
b) un antigène de détection, marqué par une enzyme, qui est un peptide de séquence (III) selon l'invention, ou une composition contenant un tel peptide.

Une autre trousse de détection d'anticorps anti VIH dans un échantillon biologique préférée comprend
a) un antigène de capture, qui est un peptide de séquence (III) selon l'invention, ledit antigène de capture étant immobilisé sur une microplaque;
b) un anticorps anti immunoglobuline marqué, notamment par une enzyme.

Les exemples qui suivent illustrent l'invention sans en limiter la portée.

### Exemple 1 : synthèse de peptides

Les synthèses ci-après ont été réalisées sur un synthétiseur Pioneer, en utilisant la chimie « Fmoc » (9-fluorénylméthyloxy carbonyl) : à chaque étape les réactifs (c'est à dire l'acide aminé protégé et les activateurs de couplage (TBTU (2 - (1H-benzotriazol-1-yl) - 1,1,3,3 - tétraméthyluronium tétrafluoroborate)/HOBt (N-hydroxybenzotriazol)) sont ajoutés en excès (dans un rapport « moles de réactifs /moles de groupes substituables sur la résine » = 5). A la fin de la synthèse, le peptide est séparé de la résine par une solution à base d'acide trifluoroacétique (réactif K). Le peptide est ensuite précipité dans une solution d'éther refroidie, puis purifié par HPLC. Sur chaque peptide linéaire synthétisé, une étape d'oxydation chimique est réalisée qui permet de cycliser le peptide au niveau des 2 cystéines.

Les inventeurs ont ainsi procédé à la synthèse des peptides cyclisés suivants :

Ce peptide de séquence SEQ ID N° 10 a été synthétisé et utilisé dans les expérimentations décrites ci-après car il représente, selon les inventeurs, le peptide de l'art antérieur le plus proche. C'est donc à ce titre qu'il est utilisé dans les comparaisons illustrées ci-après. Chacun de ces peptides a été conjugué à la peroxydase du raifort (conjugué marqué) selon la méthode bien connue de Nakane et Kawaoi (J. Histochem. Cytochem. 22, p. 1984 (1974)).

### Exemple 2 : échantillons sériques utilisés

Les sérums utilisés pour la détection d'anticorps anti-VIH sont :
- des échantillons de panels de séroconversion fournis par Boston Biomedica Inc. (7 panels ; 17 échantillons), Nabi (5 panels ; 14 échantillons) et Impath Bioclinical Partners (4 panels BCP; 10 échantillons).
- 4 échantillons sériques positifs en anticorps anti VIH-1 (C4, C5, C9 et C10) d'un panel interne de Bio-Rad, Marnes la Coquette, 92430, France
- 13 sérums de donneurs normaux et de sujets négatifs ont de plus été testés afin de déterminer la valeur-seuil des essais.

### Exemple 3 :

### Essai immunoenzymatique mettant en oeuvre les conjugués peroxydase-peptides VIH 1

La détection des anticorps dirigés contre les virus VIH est basée, dans l'exemple ci-après, sur le principe de la technique immunoenzymatique de type « sandwich double antigène ». Le test repose sur l'utilisation, d'une part, d'une microplaque (phase solide) sensibilisée avec des antigènes purifiés, dont la glycoprotéine d'enveloppe (gp160) du virus VIH 1 M, et, d'autre part, d'un conjugué constitué d'un peptide selon l'invention marqué par la peroxydase, qui prennent en sandwich un anticorps anti VIH à détecter.

Le protocole d'essai utilisé est le suivant : 100 µl de chaque échantillon sérum testé, dilué au ¾ sont distribués dans une cupule de la microplaque sensibilisée et le mélange est homogénéisé. Après incubation du mélange sous film adhésif pendant 60 minutes à 37°C, puis lavage de la microplaque à l'aide d'un tampon Tris NaCl, 100 µl de conjugué marqué (peptide VIH 1 selon l'invention marqué à la peroxydase) sont ajoutés dans chaque cupule. Le mélange est mis à incuber sous film adhésif pendant 30 minutes à 18-30°C.

Après élimination, par lavage à l'aide du tampon Tris NaCl susdit, de la fraction de conjugué restée libre, la présence de l'enzyme immobilisée sur les complexes est révélée. Pour cela, dans chaque cupule sont ajoutés 80 µl d'une solution de révélation (contenant du substrat H₂O₂ en solution d'acide citrique et d'acétate de sodium, et un chromogène, la tétraméthylbenzidine ou TMB). Après incubation du nouveau mélange à l'obscurité pendant 30 minutes à 18-30°C, la révélation est arrêtée par addition de 100 µl d'acide sulfurique 1 N. La densité optique est lue au spectrophotomètre à 450/620 nm.

La présence ou l'absence d'anticorps anti-VIH est déterminée par comparaison, pour chaque échantillon testé, de la densité optique (« DO ») enregistrée à celle de la valeur-seuil (« cut-off » en anglais) calculée (valeur-seuil = moyenne des DO d'échantillons négatifs + 0,100 unité DO).

Un échantillon est considéré comme positif (porteur d'anticorps anti-VIH) si la DO obtenue est supérieure à celle de la valeur-seuil et négatif si sa DO est inférieure à celle de la valeur-seuil.

Le tableau I montre les résultats comparatifs obtenus à l'aide des 5 conjugués fabriqués dans les mêmes conditions (couplage de chaque peptide synthétisé ci-dessus à la peroxydase). Les différents conjugués préparés sont décrits ci-après :
- Conjugué A: peptide de séquence SEQ ID N° 10 couplé à la peroxydase
- Conjugué B : peptide de SEQ ID N°1 couplé à la peroxydase
- Conjugué C : peptide de SEQ ID N°2 couplé à la peroxydase
- Conjugué D : peptide de SEQ ID N°3 couplé à la peroxydase
- Conjugué E : peptide de SEQ ID N°4 couplé à la peroxydase

Dans le tableau I ci-après, les résultats donnés sont des valeurs de DO lues après essais immunoenzymatiques réalisés suivant le protocole décrit ci-dessus et à l'aide des conjugués A, B, C, D et E décrits.

**Tableau I - Résultats exprimés en unités de densité optique**

| **Conjugué** | A | B | C | D | E |
|---|---|---|---|---|---|
| **Sérum positif** | | | | | |
| **Panel interne** | | | | | |
| C4 | 0,107 | 0,204 | 0,153 | 0,230 | 0,220 |
| C5 | 0,099 | 0,186 | 0,121 | 0,181 | 0,189 |
| C9 | 0,129 | 0,196 | 0,206 | 0,179 | 0,174 |
| C10 | 0,309 | 0,388 | 0,341 | 0,376 | 0,353 |

| **Sérum positif** | | | | | |
|---|---|---|---|---|---|
| **Panel BCP** | | | | | |
| 9017-3 | 0,025 | 0,054 | 0,044 | 0,053 | 0,044 |
| 9017-4 | 0,070 | 0,134 | 0,118 | 0,136 | 0,098 |
| 9017-5 | 0,144 | 0,254 | 0,219 | 0,229 | 0,191 |
| | | | | | |
| 9021-15 | 0,023 | 0,021 | 0,021 | 0,012 | 0,044 |
| 9021-16 | 0,139 | 0,231 | 0,187 | 0,107 | 0,098 |
| 9021-17 | 2,214 | 2,868 | 2,500 | 1,925 | 0,191 |
| | | | | | |
| 9023-21 | 0,009 | 0,013 | 0,011 | 0,010 | 0,017 |
| 9023.-22 | 0,257 | 0,404 | 0,309 | 0,402 | 0,380 |
| | | | | | |
| 9032-8 | 0,010 | 0,035 | 0,017 | 0,026 | 0,033 |
| 9032-9 | 0,244 | 0,330 | 0,252 | 0,299 | 0,314 |
| | | | | | |

| **Panel BBI** | | | | | |
|---|---|---|---|---|---|
| W9 | 0,016 | 0,023 | 0,019 | 0,017. | 0,015 |
| W10 | 2,386 | 3,093 | 2,892 | 2,748 | 2,794 |
| | | | | | |
| AQ3 | 0,017 | 0,014 | 0,015 | 0,008 | 0,012 |
| AQ4 | 0,282 | 0,322 | 0,274 | 0,295 | 0,290 |
| | | | | | |
| AT3 | 0,014 | 0,017 | 0,015 | 0,016 | 0,016 |
| AT4 | 0,082 | 0,115 | 0,092 | 0,115 | 0,105 |
| AT5 | 3,749 | 3,746 | 3,816 | 3,875 | 3,719 |
| | | | | | |
| BC3 | 0,096 | 0,078 | 0,091 | 0,090 | 0,073 |
| BC4 | 2,349 | 2,413 | 2,308 | 2,382 | 2,346 |
| | | | | | |
| BF4 | 0,007 | 0,009 | 0,009 | 0,010 | 0,007 |
| BF5 | 0,148 | 0,196 | 0,170 | 0,200 | 0,190 |
| | | | | | |
| BG5 | 0,008 | 0,011 | 0,009 | 0,009 | 0,010 |
| BG6 | 0,200 | 0,289 | 0,234 | 0,266 | 0,278 |
| BG7 | 3,455 | 3,720 | 3,540 | 3,730 | 3,674 |
| | | | | | |
| BI1 | 0,008 | 0,010 | 0,010 | 0,010 | 0,009 |
| BI2 | 0,068 | 0,122 | 0,088 | 0,121 | 0,105 |
| BI3 | 1,642 | 1,494 | 1,607 | 1,598 | 1,563 |

| **Panel NABI** | | | | | |
|---|---|---|---|---|---|
| 241-B | 0,063 | 0,080 | 0,067 | 0,076 | 0,077 |
| 241-C | 0,070 | 0,114 | 0,099 | 0,114 | 0,110 |
| 241-D | 3,838 | 3,867 | 3,795 | 3,770 | 3,745 |
| | | | | | |
| 271-C | 0,022 | 0,047 | 0,037 | 0,030 | 0,023 |
| 271-D | 0,637 | 1,304 | 0,975 | 0,463 | 0,499 |
| | | | | | |
| 401-C | 0,008 | 0,012 | 0,010 | 0,010 | 0,009 |
| 401-D | 0,091 | 0,275 | 0,164 | 0,231 | 0,247 |
| 401-E | 1,395 | 2,226 | 1,784 | 2,263 | 2,333 |
| | | | | | |
| 407-D | 0,007 | 0,011 | 0,010 | 0,010 | 0,011 |
| 407-E | 0,105 | 0,121 | 0,110 | 0,090 | 0,087 |
| 407-F | 3,923 | 4,000 | 3,830 | 4,000 | 4,000 |
| | | | | | |
| 409-B | 0,007 | 0,010 | 0,009 | 0,007 | 0,007 |
| 409-C | 0,091 | 0,146 | 0,114 | 0,141 | 0,144 |
| 409-D | 1,334 | 1,811 | 1,528 | 1,889 | 1,868 |
| | | | | | |

| **Conjugué** | A | B | C | D | E |
|---|---|---|---|---|---|
| **Sérums négatifs** | | | | | |
| **N = 13** | | | | | |
| Moyenne | 0,008 | 0,009 | 0,009 | 0,010 | 0,009 |
| Valeur-seuil | 0,108 | 0,109 | 0,109 | 0,110 | 0,109 |

Les conjugués B, C, D et E obtenus avec des peptides selon l'invention donnent, pour les échantillons positifs testés, et à valeur-seuil quasi identique, une valeur de DO significativement supérieure à celle obtenue avec le conjugué A du peptide 35mer de l'art antérieur (peptide de séquence (SEQ ID N° 10).

Dans le tableau II ci-après, les mêmes résultats sont exprimés en termes de rapport DO échantillon/valeur-seuil (DONS).

**Tableau II - Résultats exprimés en rapport Densité optique/valeur-seuil**

| **Conjugué** | A | B | C | D | E |
|---|---|---|---|---|---|
| **Sérum positif** | | | | | |
| **Panel interne** | | | | | |
| C4 | 0,99 | 1,87 | 1,39 | 2,09 | 2,02 |
| C5 | 0,92 | 1,71 | 1,10 | 1,65 | 1,73 |
| C9 | 1,19 | 1,80 | 1,87 | 1,63 | 1,60 |
| C10 | 2,86 | 3,56 | 3,10 | 3,42 | 3,24 |

| **Sérum positif** | | | | | |
|---|---|---|---|---|---|
| **Panel BCP** | | | | | |
| 9017-3 | 0,23 | 0,50 | 0,40 | 0,48 | 0,40 |
| 9017-4 | 0,65 | 1,23 | 1,07 | 1,24 | 0,90 |
| 9017-5 | 1,33 | 2,33 | 1,99 | 2,08 | 1,75 |
| | | | | | |
| 9021-15 | 0,21 | 0,19 | 0,19 | 0,11 | 0,19 |
| 9021-16 | 1,29 | 2,12 | 1,70 | 0,97 | 1,06 |
| 9021-17 | 20,50 | 26,31 | 22,73 | 1,50 | 17,92 |
| | | | | | |
| 9023-21 | 0,08 | 0,12 | 0,10 | 0,09 | 0,16 |
| 9023.-22 | 2,38 | 3,71 | 2,81 | 3,65 | 3,49 |
| | | | | | |
| 9032-8 | 0,09 | 0,32 | 0,16 | 0,24 | 0,30 |
| 9032-9 | 2,26 | 3,03 | 2,29 | 2,72 | 2,88 |

| **Panel BBI** | | | | | |
|---|---|---|---|---|---|
| W9 | 0,15 | 0,21 | 0,18 | 0,15 | 0,14 |
| W10 | 26,26 | 28,38 | 26,29 | 24,98 | 25,63 |
| | | | | | |
| AQ3 | 0,16 | 0,13 | 0,14 | 0, 07 | 0,11 |
| AQ4 | 2,61 | 2,95 | 2,49 | 2,68 | 2,66 |
| | | | | | |
| AT3 | 0,13 | 0,16 | 0,13 | 0,15 | 0,15 |
| AT4 | 0,76 | 1,06 | 0,84 | 1,05 | 0,96 |
| AT5 | 34,71 | 34,37 | 34,69 | 35,23 | 34,12 |
| | | | | | |
| BC3 | 0,89 | 0,72 | 0,82 | 0,82 | 0,67 |
| BC4 | 21,75 | 22,14 | 20,98 | 21,65 | 21,52 |
| | | | | | |
| BF4 | 0,06 | 0,08 | 0,08 | 0,09 | 0,06 |
| BF5 | 1,37 | 1,80 | 1,54 | 1,82 | 1,74 |
| BG5 | 0,07 | 0,10 | 0,08 | 0,08 | 0,09 |
| BG6 | 1,85 | 2,65 | 2,13 | 2,42 | 2,55 |
| BG7 | 31,99 | 34,13 | 32,18 | 33,91 | 33,71 |
| | | | | | |
| BI1 | 0,07 | 0,09 | 0,09 | 0,09 | 0,08 |
| BI2 | 0,63 | 1,12 | 0,80 | 1,10 | 0,96 |
| BI3 | 15,20 | 13,71 | 14,61 | 14,53 | 14,34 |

| **Panel NABI** | | | | | |
|---|---|---|---|---|---|
| 241-B | 0,58 | 0,73 | 0,60 | 0,69 | 0,71 |
| 241-C | 0,65 | 1,05 | 0,90 | 1,04 | 1,01 |
| 241-D | 35,54 | 35,48 | 34,50 | 34,27 | 34,36 |
| | | | | | |
| 271-C | 0,20 | 0,43 | 0,34 | 0,27 | 0,21 |
| 271-D | 5,90 | 11,96 | 8,86 | 4,21 | 4,58 |
| | | | | | |
| 401-C | 0,07 | 0,11 | 0,09 | 0,09 | 0,08 |
| 401-D | 0,84 | 2,52 | 1,49 | 2,10 | 2,27 |
| 401-E | 12,92 | 20,42 | 16,21 | 20,57 | 21,40 |
| | | | | | |
| 407-D | 0,06 | 0,10 | 0,09 | 0,09 | 0,10 |
| 407-E | 0,97 | 1,11 | 1,00 | 0,82 | 0,80 |
| 407-F | 36,32 | 36,70 | 34,82 | 36,36 | 36,70 |
| | | | | | |
| 409-B | 0,06 | 0,09 | 0,08 | 0,06 | 0,06 |
| 409-C | 0,84 | 1,34 | 1,03 | 1,28 | 1,32 |
| 409-D | 12,35 | 16,61 | 13,89 | 17,17 | 17,14 |

A valeur-seuil quasi identique, les conjugués B, C, D et E obtenus avec des peptides selon l'invention donnent, pour les échantillons testés, des rapports DO/VS significativement supérieurs à ceux obtenus avec le conjugué A du peptide 35mer de l'art antérieur (peptide de séquence SEQ ID N° 10). De même, certains échantillons négatifs avec le conjugué A du peptide de l'art antérieur sont trouvés positifs avec les peptides selon l'invention.

Le tableau des résultats exprimés en rapport DONS (tableau III) montre la répartition des échantillons en fonction des rapports DONS obtenus :

**Tableau III**

| **Conjugué** | A | B | C | D | E |
|---|---|---|---|---|---|
| **Sérums positifs** | | | | | |
| **N =45** | | | | | |
| Rapport DO/VS < 1 | 25 | 16 | 19 | 19 | 20 |
| Rapport DO/VS > 1 | 20 | 29 | 26 | 26 | 25 |

Le Tableau III montre clairement que les sérums positifs testés sont mieux détectés avec les peptides selon l'invention qu'avec le peptide de l'art antérieur de séquence SEQ ID N° 10 (conjugué A), car le nombre d'échantillons donnant un rapport DO/VS supérieur à 1 est significativement plus grand.

Comme le montrent globalement les tableaux I, II et III, les peptides selon l'invention (conjugués B, C, D et E de séquence (III)) permettent de manière significative de détecter plus précocément les séroconversions que le peptide de l'art antérieur de séquence SEQ ID N° 10 (conjugué A).

Dans le tableau IV ci-après, l'immunoréactivité des conjugués B, C, D et E est donnée en pourcentage de l'immunoréactivité du conjugué A qui représente la référence.

**Tableau IV - Résultats exprimés en pourcentage d'immunoréactivité par rapport au conjugué A**

| **Conjugué** | B | C | D | E |
|---|---|---|---|---|
| | | | | |
| **Sérum positif** | | | | |
| **Panel interne** | | | | |
| C4 | 191 % | 133 % | 215 % | 206 % |
| C5 | 188 % | 113 % | 183 % | 191 % |
| C9 | 152 % | 168 % | 139 % | 135 % |
| C10 | 126% | 109% | 122% | 114% |

| **Sérum positif** | | | | |
|---|---|---|---|---|
| **Panel BCP** | | | | |
| 9017-3 | 216 % | 180 % | 212 % | 176 % |
| 9017-4 | 191 % | 161 % | 194 % | 140 % |
| 9017-5 | 176 % | 150 % | 159 % | 133 % |
| | | | | |
| 9021-15 | 91 % | 95 % | 52 % | 91 % |
| 9021-16 | 166 % | 129 % | 77 % | 83 % |
| 9021-17 | 130 % | 109 % | 87 % | 88 % |
| | | | | |
| 9023-21 | 144 % | 108 % | 111 % | 189 % |
| 9023.-22 | 157 % | 111 % | 156 % | 148 % |
| | | | | |
| 9032-8 | 350 % | 158 % | 260 % | 330 % |
| 9032-9 | 135 % | 102 % | 123 % | 129 % |

| **Panel BBI** | | | | |
|---|---|---|---|---|
| W9 | 144% | 113% | 106% | 94 % |
| W10 | 109 % | 101 % | 97 % | 99 % |
| | | | | |
| AQ3 | 82 % | 96 % | 47 % | 71 % |
| AQ4 | 114 % | 94 % | 105 % | 103 % |
| | | | | |
| AT3 | 121 % | 107 % | 114 % | 114 % |
| AT4 | 140 % | 109 % | 140 % | 128 % |
| AT5 | 100% | 101 % | 103 % | 99 % |
| | | | | |
| BC3 | 81 % | 98 % | 94 % | 76 % |
| BC4 | 103 % | 98 % | 101 % | 100 % |
| | | | | |
| BF4 | 129% | 119% | 143 % | 100% |
| BF5 | 132 % | 111 % | 135 % | 128 % |
| | | | | |
| BG5 | 138 % | 112 % | 113 % | 125 % |
| BG6 | 145 % | 113 % | 133 % | 139 % |
| BG7 | 108 % | 101 % | 108 % | 106 % |
| | | | | |
| BI1 | 125% | 133 % | 125% | 113% |
| BI2 | 179% | 120% | 178% | 154% |
| BI3 | 91 % | 98 % | 97 % | 95 % |

| **Panel NABI** | | | | |
|---|---|---|---|---|
| 241-B | 127 % | 99 % | 121 % | 122 % |
| 241-C | 163 % | 129 % | 163 % | 157 % |
| 241-D | 101 % | 98 % | 98 % | 98 % |
| | | | | |
| 271-C | 214 % | 140 % | 136 % | 105 % |
| 271-D | 205 % | 139 % | 73 % | 98 % |
| | | | | |
| 401-C | 150% | 117% | 125% | 113% |
| 401-D | 302 % | 166 % | 254 % | 271 % |
| 401-E | 160 % | 121 % | 162 % | 167 % |
| | | | | |
| 407-D | 157 % | 133 % | 143 % | 157 % |
| 407-E | 115 % | 105 % | 86 % | 83 % |
| 407-F | 102 % | 97 % | 102 % | 102 % |
| | | | | |
| 409-B | 143 % | 120 % | 100 % | 100 % |
| 409-C | 160 % | 118 % | 155 % | 158 % |
| 409-D | 136 % | 110 % | 142 % | 140 % |

Le tableau IV montre bien que les peptides selon l'invention (conjugués B, C, D et E de séquence (III)) présentent une immunoréactivité en général significativement supérieure à celle du peptide de l'art antérieur de séquence SEQ ID N° 10 et donc présentent une sensibilité de détection généralement plus élevée.

En résumé, tous les résultats ci-dessus montrent que les sérums positifs en anticorps anti VIH1 testés sont détectés par les peptides selon l'invention (conjugués B, C, D et E de séquence (III)) de façon significativement supérieure à la détection obtenue par un peptide de l'art antérieur (conjugué A de séquence SEQ ID N° 10), le peptide le plus proche. De plus, les peptides selon l'invention (conjugués B, C, D et E de séquence (III)) permettent de détecter encore plus précocément les séroconversions.

L'invention répond donc bien, au moyen des peptides selon l'invention, au problème posé.

## Revendications

1. Peptide cyclique de la gp41 du virus VIH-1 de séquence (III) : où
X représente un acide aminé choisi dans le groupe constitué par F et G,
et
où les deux acides aminés aa₁ et aa₂ soit sont absents, soit représentent
aa₁ : l'alanine
aa₂ : la sérine.

2. Peptide selon la revendication 1 choisi dans le groupe constitué par les peptides suivants :

3. Composition antigénique comprenant un peptide selon la revendication 1 ou la revendication 2.

4. Procédé de préparation d'un peptide de séquence (III), tel que défini à la revendication 1 ou à la revendication 2, ledit procédé consistant en la synthèse chimique d'un tel peptide.

5. Procédé de détection in vitro d'anticorps anti VIH-1 dans un échantillon biologique comprenant les étapes consistant en :
a) la mise en contact de l'échantillon biologique avec au moins un peptide de la gp41 du virus VIH-1 de séquence (III), tel que défini à la revendication 1 ou à la revendication 2, et/ou une composition selon la revendication 3;
b) l'incubation du mélange dans des conditions permettant la formation de complexes antigène - anticorps entre le ou les peptides de séquence (III) et des anticorps anti-VIH-1 présents dans l'échantillon biologique ; et
c) la révélation et la détection des complexes antigènes-anticorps formés.

6. Procédé de détection selon la revendication 5, dans lequel l'étape a) comprend la mise en contact de l'échantillon biologique avec une composition antigénique constituée d'au moins un peptide de la gp41 de séquence (III), et d'un antigène du VIH-2 et/ou d'un antigène du VIH-1 O.

7. Trousse de détection d'anticorps anti VIH dans un échantillon biologique, ladite trousse contenant un peptide de la gp41 du virus VIH1 de séquence (III), tel que défini à la revendication 1 ou à la revendication 2, ou une composition selon la revendication 3, éventuellement en association avec un antigène du VIH-2 et/ou un antigène du VIH-1 O.

8. Utilisation d'un peptide de la gp41 du virus VIH1 de séquence (III), tel que défini à la revendication 1 ou à la revendication 2, ou une composition selon la revendication 3, pour la détection d'anticorps anti-VIH dans un échantillon biologique.

## Claims

1. Cyclic peptide of HIV-1 virus gp41 with sequence (III):
RILAVERYLKDQQLLGIWGC₆₀₃SGKLIC₆₀₉TTAVPX₆₁₅Naa₁aa₂ (III)
where
X represents an amino acid selected from the group formed by F and G, and
where both amino acids aa₁ and aa₂ are either absent or represent
aa₁: alanine
aa₂: serine.

2. Peptide according to claim 1 selected from the group made up of the following peptides:
SEQ ID No.1: RILAVERYLKDQQLLGIWGCSGKLICTTAVPF₆₁₅NA₆₁₇S₆₁₈
SEQ ID No.2: RILAVERYLKDQQLLGIWGCSGKLICTTAVPF₆₁₅N
SEQ ID No.3: RILAVERYLKDQQLLGIWGCSGKLICTTAVPG₆₁₅N
SEQ ID No.4: RILAVERYLKDQQLLGIWGCSGKLICTTAVPG₆₁₅NA₆₁₇S₆₁₈

3. Antigenic composition which includes a peptide according to claim 1 or claim 2.

4. Process for preparing a peptide with sequence (III), as identified in claim 1 or claim 2, where the said procedure involves the synthesis of such a peptide.

5. In-vitro detection process for anti-HIV antibody in a biological sample which includes the steps involving:
a) bringing the biological sample into contact with at least one peptide from the HIV-1 virus gp41 with sequence (III), as defined in claim 1 or in claim 2, and/or a composition according to claim 3 ;
b) incubation of the mixture under conditions which allow antigen - antibody complexes to form between the peptide or peptides with sequence (III) and anti HIV-1 antibodies present in the biological sample; and
c) the revelation and detection of the antigen-antibody complexes formed.

6. Procedure for detection according to claim 5 in which step a) includes bringing the biological sample into contact with an antigen composition made up of at least one gp41 peptide with the sequence (III), and of a HIV-2 antigen and/or an HIV-1 O Antigen.

7. Kit for detecting anti-HIV antibody in a biological sample where the said kit contains a peptide from the HIV-1 virus gp41 with a sequence (III), as defined in claim 1 or in claim 2, or a composition according to claim 3, possibly in combination with a HIV-2 antigen and/or a HIV-1 O antigen.

8. The use of a peptide from the HIV-1 virus gp41 with a sequence (III), as defined in claim 1 or in claim 2, or a composition according to claim 3, for the detection of an anti-HIV antibody in a biological sample.

## Patentansprüche

1. Cyclisches Peptid des gp41 des HIV-1-Virus mit der Sequenz (III) :
RILAVERYLKDQQLLGIWGC₆₀₃SGKLIC₆₀₉TTAVPX₆₁₅Naa₁aa₂ (III),
worin
X eine Aminosäure bedeutet, die aus der Gruppe ausgewählt ist, die von F und G gebildet wird, und
die zwei Aminosäuren aa₁ und aa₂ entweder fehlen oder
aa₁: Alanin
aa₂: Serin
bedeuten.

2. Peptid nach Anspruch 1, das aus der Gruppe ausgewählt ist, die von folgenden Peptiden gebildet wird:
SEQ ID Nr. 1: RILAVERYLKDQQLLGIWGCSGKLICTTAVPF₆₁₅NA₆₁₇S₆₁₈
SEQ ID Nr. 2: RILAVERYLKDQQLLGIWGCSGKLICTTAVPF₆₁₅N
SEQ ID Nr.3: RILAVERYLKDQQLLGIWGCSGKLICTTAVPG₆₁₅N
SEQ ID Nr. 4: RILAVERYLKDQQLLGIWGCSGKLICTTAVPG₆₁₅N A₆₁₇S₆₁₈.

3. Antigenzusammensetzung, die ein Peptid nach Anspruch 1 oder 2 umfasst.

4. Verfahren zur Herstellung eines in Anspruch 1 oder 2 definierten Peptids mit der Sequenz (III), wobei das Verfahren in der chemischen Synthese eines solchen Peptids besteht.

5. Verfahren zum in-vitro-Detektieren von Anti-HIV-1-Antikörpern in einer biologischen Probe, das die Stufen umfasst, die bestehen aus dem:
a) In-Berührung-Bringen der biologischen Probe mit mindestens einem Peptid des gp41 des HIV-1-Virus mit der Sequenz (III), wie in Anspruch 1 oder 2 definiert, und/oder einer Zusammensetzung nach Anspruch 3,
b) Inkubieren des Gemischs unter Bedingungen, welche die Bildung von Antigen-Antikörper-Komplexen zwischen dem/den Peptid/en mit der Sequenz (III) und den in der biologischen Probe vorhandenen Anti-HIV-1-Antikörpern erlauben, und
c) Nachweisen und Detektieren der gebildeten Antigen-Antikörper-Komplexe.

6. Detektionsverfahren nach Anspruch 5, in welchem die Stufe a) das In-Berührung-Bringen der biologischen Probe mit einer Antigenzusammensetzung umfasst, die aus mindestens einem Peptid des gp41 mit der Sequenz (III) und einem Antigen des HIV-2 und/oder einem Antigen des HIV-1 O besteht.

7. Kit zur Detektion von Anti-HIV-Antikörpern in einer biologischen Probe, wobei der Kit ein Peptid des gp41 des HIV-1-Virus mit der Sequenz (III), wie in Anspruch 1 oder 2 definiert, oder eine Zusammensetzung nach Anspruch 3, gegebenenfalls zusammen mit einem Antigen des HIV-2 und/oder einem Antigen des HIV-1 O, enthält.

8. Verwendung eines Peptids des gp41 des HIV-1-Virus mit der Sequenz (III), wie in Anspruch 1 oder 2 definiert, oder einer Zusammensetzung nach Anspruch 3 für die Detektion von Anti-HIV-Antikörpern in einer biologischen Probe.
